# EUROPEAN PATENT SPECIFICATION

(11) **EP 4 292 633 B1**
(45) Date of publication and mention of the grant of the patent: **04.06.2025**
(21) Application number: 22795967.3
(22) Date of filing: 23.03.2022
(51) Int. Cl.: A61M 16/08, A61M 16/10, A61M 16/14

(54) **VENTILATOR MEDICATION INJECTION STRUCTURE**
STRUKTUR ZUR INJEKTION VON MEDIKAMENTEN IN EINEM BEATMUNGSGERÄT
STRUCTURE D'INJECTION DE MÉDICAMENT POUR VENTILATEUR

(30) Priority: 26.04.2021 KR 20210053445
(43) Date of publication of application: 20.12.2023
(73) Proprietor: Boditech Med Inc., Chuncheon-si, Gangwon-do 24398 (KR)
(72) Inventor: CHOI, Doo Young, Gwangmyeong-si Gyeonggi-do 14318 (KR)
(74) Representative: Ter Meer Steinmeister & Partner
(86) International application number: PCT/KR2022/004024
(87) International publication number: WO 2022/231133

(56) References cited:
- EP-B1- 0 758 257
- KR-A- 20080 017 375
- KR-A- 20110 034 633
- KR-A- 20190 030 976
- KR-B1- 101 311 751
- KR-B1- 101 424 934
- US-A- 4 953 547
- US-A- 5 181 508
- US-A- 5 207 220
- US-A- 5 947 120
- US-B2- 7 600 511

## Description

### Technical Field

The present disclosure relates to a ventilator medication injection structure mounted on a hose path connecting a ventilator to a patient's mask so that medication is capable of being included in air conditioned by the ventilator, thereby being capable of providing the medication to the patient.

### Background Art

Generally, a patient who has difficulty in spontaneous breathing relies on a ventilator to breathe. In addition, medication may be prescribed according to a decision of a doctor, and the medication may be included in air conditioned in a ventilator and then provided to a patient. Various medications may be used according to a patient's physical condition and, for example, medication that helps a lung function may be used when a patient's lung function is bad.

Conventionally, in order to inject medication, a T-tube is disposed on a tube that is connected to a mask or a mouthpiece from a ventilator, a medication case is mounted on one side of an inlet of the T-tube, and the medication is injected into the medication case. In addition, an open portion of the medication case is covered with a lid so as to prevent contamination.

The T-tube is provided with a medication vaporizing apparatus, and the medication is vaporized using the medication vaporizing apparatus and the vaporized medication is included in conditioned air, and then the vaporized medication is provided to a patient.

However, as described above, a conventional technology has problems such as contamination, labor, cost, and so on. More specifically, although a timing of medication injection varies according to a prescription of a doctor, the timing may be set such that medication is injected approximately two to four times a day, and the lid is then removed and closed each time. In this process, there are problems in that a time for which the medication case is exposed is prolonged inevitably, the possibility of contamination may increase, there is the hassle of opening and closing the lid, and the cost increases since a tube replacement cycle is fast as the possibility of contamination is high.

Meanwhile, the ventilator is configured to purify air, to heat the air to the temperature similar to body temperature, to appropriately adjust the pressure of the air, and to provide the air that is conditioned to a patient. However, the temperature of the conditioned air may be changed while the conditioned air is moved along the tube, and there is a problem that the air having a low temperature is provided to a patient's respiratory organs.

### [Documents of Related Art]

(Patent Document 1) KR 10-2007-0106964 A
(Patent Document 2) KR 10-1137052 B2
(Patent Document 3) KR 10-2007-0004058 A
(Patent Document 4) KR 10-2010-0060004 A
(Patent Document 5) KR 10-1459399 B1

Some prior art medication injection structures for use in ventilation systems are known from the documents US 5 181 508 A, EP 0 758 257 B1, US 5 947 120 A and US 4 953 547 A.

The document US 5 181 508 A, for example, discloses a drug administering endotracheal respiration system for administering vital life-saving drugs into the lungs of a victim while maintaining the flow of life-supporting gas thereto via ventilation apparatus comprising in combination: a gas supply; a tube for establishing gas flow exchange between the lungs of the victim and the gas supply, the tube having a proximal end and a distal end for insertion into the trachea of the victim; and a connector for coupling the proximal end of the tube to the gas supply, the connector being formed as a cylinder with a gas input end and a gas discharge end and a linear main axial passageway therebetween, the connector having a first port adapted for injecting vital life-saving drugs in liquid form into the passageway and then into the distal end of the tube intermixed with the gas, the first port having a portion integrally formed with the connector and a portion separable therefrom, the connector further having a second port adapted for injecting vital life-saving drugs in liquid form into the passageway and then into the distal end of the tube intermixed with the gas, the second port having a portion integrally formed with the connector and a portion separable therefrom, the main axial passageway adapted for atomizing and intermixing of the flow of life-saving drugs with the life-supporting gas. At least one port is a hypodermic needle port with a one way rubber member through which a needle is advanced and retracted, or a syringe port with a one way, spring loaded, resealable valve opened and closed by the insertion and removal of syringe.

### Disclosure

### Technical Problem

Accordingly, the present disclosure has been made keeping in mind the above problems occurring in the related art, and an objective of the present disclosure is to provide a ventilator medication injection structure capable of minimizing an exposure of an inner portion of a medication case when medication is injected, the structure being capable of preventing the medication case from being contaminated, and the structure being configured such that a patient can live in a more strictly clean environment.

Another objective of the present disclosure is to provide a ventilator medication injection structure configured to reheat air conditioned in a ventilator when the temperature of the conditioned air becomes lower than an appropriate temperature while the conditioned air moving along a tube although the conditioned air has been heated to the appropriate temperature, thereby being capable of providing the conditioned air having the appropriate temperature to a patient.

### Technical Solution

In order to achieve the above objectives, according to an embodiment of the present disclosure, there is provided a ventilator medication injection structure including: a T-tube 10 having a tube body extending from an inlet 11 to an outlet 12, with a branch tube 13 being formed on one side of the tube body; a cover 20 having a first tube body 22 formed below a flange 21, a second tube body 23 formed inside the first tube body 22 such that the second tube body 23 penetrates the flange 21, and a male screw 24 formed on an outer peripheral surface of the second tube body 23; and a packing closure 30 having a third tube body 32 formed at a center of a body 31 such that the third tube body 32 is fitted inside the second tube body 23, a fourth tube body 33 formed on an outside of the third tube body 32, a female screw 34 formed on the fourth tube body 33, the female screw 34 being fastened to the male screw 24, and a packing 36 which has elasticity and which is disposed inside the body 31.

The first tube body 22 is fitted and assembled to the branch tube 13, and medication is capable of being injected inside the T-tube 10 with a syringe by piercing the packing 36 with a syringe needle and passing the syringe needle through the third tube body 32.

In addition, in the ventilator medication injection structure according to an embodiment of the present disclosure, the first tube body 22 may be provided in a tapered shape having a narrow lower side, and may be formed of a material having elasticity.

In addition, in the ventilator medication injection structure according to an embodiment of the present disclosure, a power connector 14 to which an external power source is connected may be disposed on one side of the T-tube 10, a heating wire 15 may be disposed inside the T-tube 10, and the heating wire 15 may be configured to be connected to the external power source and is configured to heat conditioned air.

Details of other embodiments are included in the detailed description and drawings.

### Advantageous Effects

In the ventilator medication injection structure according to an embodiment of the present disclosure, medication is capable of being injected into the T-tube 10 while the syringe needle is inserted into the packing 36, and the packing 36 is configured such that a space through which the syringe needle was passed is blocked when the syringe needle is removed from the packing 36, so that an inner portion of the T-tube 10 is capable of being fundamentally prevented from being exposed.

Accordingly, in the ventilator medication injection structure according to an embodiment of the present disclosure, an exposure of an inner portion of the medication case may be minimized or the exposure of the inner portion of the medication case may be fundamentally prevented, so that the medication case is capable of being prevented from being contaminated. Furthermore, a patient can live in a more strictly clean environment.

Therefore, in the ventilator medication injection structure according to an embodiment of the present disclosure, even when the temperature of conditioned air to be provided to a patient is low, the conditioned air is capable of being reheated, so that the patient can always breathe the conditioned air having an appropriate temperature.

### Description of Drawings

FIG. 1 to FIG. 3 are views illustrating a ventilator medication injection structure according to an embodiment of the present disclosure, in which FIG. 1 is a view illustrating an external appearance of the ventilator medication injection structure, FIG. 2 is an exploded cross-sectional view in which elements of the ventilator medication injection structure are disassembled, and FIG. 3 is a cross-sectional view in which the elements are assembled.

FIG. 4 is a view illustrating a cover in the ventilator medication injection structure according to an embodiment of the present disclosure.

### Best Mode

The attached drawings for illustrating exemplary embodiments of the present disclosure are referred to in order to gain a sufficient understanding of the present disclosure, the merits thereof, and the objectives accomplished by the implementation of the present disclosure.

Hereinafter, embodiments of the present disclosure will be described in detail with reference to the accompanying drawings. It should be understood that the embodiments described below are illustratively shown to aid understanding of the present disclosure, and that the present disclosure may be implemented with various modifications different from the embodiments described herein. However, when it is determined that a detailed description of a related well-known function or component may unnecessarily obscure the gist of the present disclosure while describing the present disclosure, the detailed description and specific illustration thereof will be omitted. In addition, the accompanying drawings may not be drawn to scale, but the size of some components may be exaggerated to aid in understanding of the present disclosure.

Terms such as 'first' and 'second' are used herein merely to describe a variety of constituent elements, but the constituent elements are not limited by the terms. The terms are used only for the purpose of distinguishing one constituent element from another constituent element. For example, a first element may be termed a second element, and a second element may be termed a first element, without departing from the scope of the present disclosure.

The terms used in the specification are defined in consideration of functions used in the present disclosure, and can be changed according to the intent or conventionally used methods of producers. Accordingly, definitions of the terms should be understood on the basis of the entire description of the present specification.

Like reference numerals refer to like elements throughout the specification.

### [Description of Reference Numerals]

10: T-tube 11: inlet
12: outlet 13: branch tube
14: power connector 15: heating wire
20: cover 21: flange
22, 23: first and second tube bodies 24: male screw
30: packing closure 31: body
32, 33: third and fourth tube bodies 34: female screw
35: pocket 36: packing

### Mode for Invention

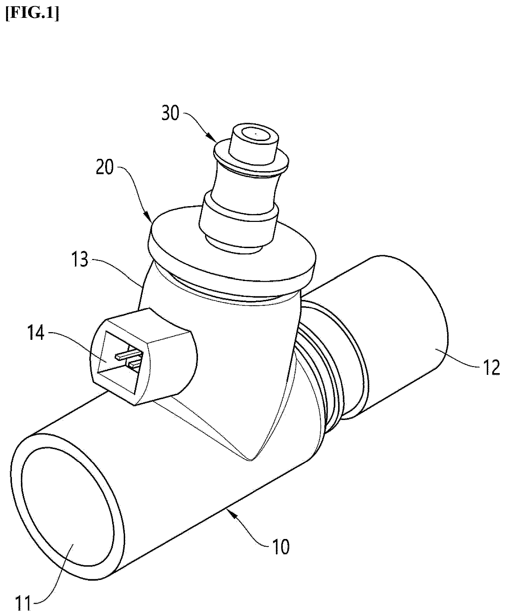

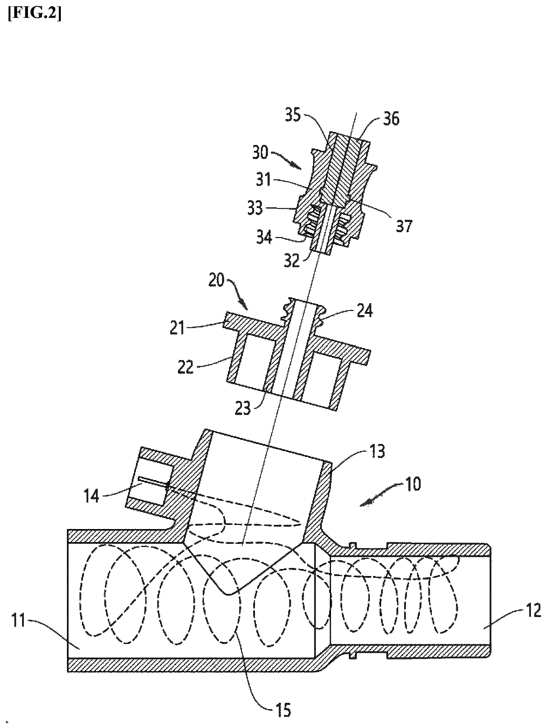

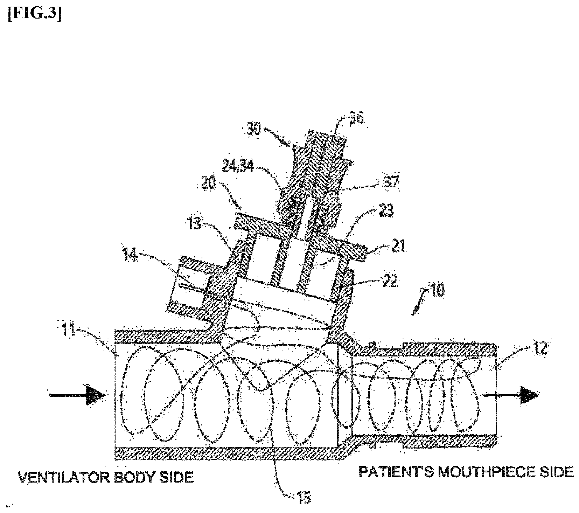

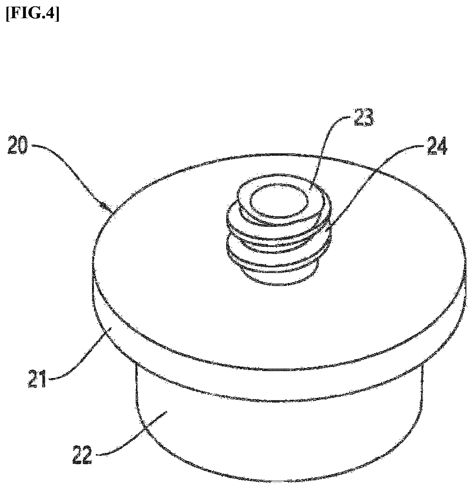

Hereinafter, referring to FIG. 1 to FIG. 4, a ventilator medication injection structure according to an embodiment of the present disclosure will be described. FIG. 1 to FIG. 3 are views illustrating a ventilator medication injection structure according to an embodiment of the present disclosure, in which FIG. 1 is a view illustrating an external appearance of the ventilator medication injection structure, FIG. 2 is an exploded cross-sectional view in which elements of the ventilator medication injection structure are disassembled, and FIG. 3 is a cross-sectional view in which the elements are assembled. FIG. 4 is a view illustrating a cover in the ventilator medication injection structure according to an embodiment of the present disclosure.

A ventilator medication injection structure according to an embodiment of the present disclosure may include a T-tube 10, a cover 20, and a packing closure 30.

As illustrated in FIG. 1 to FIG. 3, in the T-tube 10, a tube body extends from an inlet 11 to an outlet 12, and a branch tube 13 is formed on one side of the tube body.

As illustrated in FIG. 2, in the cover 20, a first tube body 22 is formed below a flange 21, and a second tube body 23 is formed inside the first tube body 22 such that the second tube body 23 penetrates the flange 21. A male screw 24 is formed on an outer peripheral surface of the second tube body 23.

As illustrated in FIG. 3, the first tube body 22 is fitted and assembled to the branch tube 13.

In the packing closure 30, a third tube body 32 is formed at a center of a body 31 such that the third tube body 32 is fitted inside the second tube body 23. A fourth tube body 33 is formed on an outside of the third tube body 32, and a female screw 34 is formed on the fourth tube body 33. As illustrated in FIG. 3, the female screw 34 is fastened to the male screw 24.

In addition, a pocket 35 is formed inside the body 31, and the pocket 35 may be densely filled with a packing 36.

The packing 36 has elasticity. More specifically, the packing 36 may be formed of soft rubber. Accordingly, when a syringe needle pierces the packing 36, the syringe needle may penetrate the packing 36. Furthermore, when the syringe needle is pulled out, a piercing made by the syringe needle is immediately filled by the elasticity of the packing 36.

Medication may be injected inside the T-tube 10 with a syringe by piercing the packing 36 with the syringe needle and passing the syringe needle through the third tube body 32.

In the ventilator medication injection structure according to an embodiment of the present disclosure, medication is capable of being injected into the T-tube 10 while a syringe needle is inserted into the packing 36, and the packing 36 is configured such that a space through which the syringe needle was passed is blocked when the syringe needle is removed from the packing 36, so that an inner portion of the T-tube 10 is capable of being fundamentally prevented from being exposed.

Accordingly, in the ventilator medication injection structure according to an embodiment of the present disclosure, the exposure of the inner portion of the T-tube 10 that serves as a medication case may be minimized or the medication case may be fundamentally blocked from being exposed, so that contamination of the medication case is prevented. Furthermore, a patient can live in a more strictly clean environment.

In addition, by preventing the exposure of the inner portion of the T-tube 10, an entire section of the tube connected from the ventilator to a mask or a mouthpiece may be strictly prevented from being exposed to the outside.

Meanwhile, as illustrated in FIG. 4, the first tube body 22 may be provided in a tapered shape having a narrow lower side, and may be formed of a material having elasticity. Accordingly, as illustrated in FIG. 2 and FIG. 3, when the ventilator medication injection structure in a disassembled state is assembled, the cover 20 is capable of being fitted and assembled in a straight direction without rotating the cover 20.

Since the first tube body 22 is provided in the tapered shape, the first tube body 22 is easily assembled due to a large diameter difference at the beginning of assembly when the first tube body 22 is inserted into the branch tube 13, and the first tube body 22 may be assembled more and more tightly as the assembly progresses.

In addition, since the first tube body 22 has an elastic property, the first tube body 22 is in close contact with an entire of inner peripheral surface of the branch tube 13, so that contaminants from the outside may be fundamentally prevented from being introduced into the inner portion of the T-tube 10.

On the other hand, as illustrated in FIG. 1 to FIG. 3, a power connector 14 to which an external power source is capable of being connected is disposed on one side of the T-tube 10.

As illustrated in FIG. 2 and FIG. 3, a heating wire 15 may be disposed inside the T-tube 10, the heating wire 15 may generate heat by being connected to the external power source, and air conditioned as described above may be heated by the heat.

Therefore, in the ventilator medication injection structure according to an embodiment of the present disclosure, even when the temperature of conditioned air to be provided to a patient is low, the conditioned air is capable of being reheated, and the patient can always breathe the conditioned air at an appropriate temperature.

Although an exemplary embodiment of the present disclosure was described above with reference to the accompanying drawings, those skilled in the art would understand that the present disclosure may be implemented in various ways without departing from the scope of the prevent disclosure.

Therefore, it should be understood that the embodiment described above is not limitative, but only an example in all respects, the scope of the present disclosure is expressed by claims described below, not the detailed description, and it should be construed that all of changes and modifications achieved from the meanings and scope of claims and equivalent concept are included in the scope of the present disclosure.

### Industrial Applicability

A ventilator medication injection structure according to an embodiment of the present disclosure may be used for including medication in conditioned air and for heating the conditioned air when the conditioned air that is conditioned from a ventilator is provided to a patient.

## Claims

1. A ventilator medication injection structure comprising:
a T-tube (10) having a tube body extending from an inlet (11) to an outlet (12), with a branch tube (13) being formed on one side of the tube body;
a cover (20) having a first tube body (22) formed below a flange (21), a second tube body (23) formed inside the first tube body (22) such that the second tube body (23) penetrates the flange (21), and a male screw (24) formed on an outer peripheral surface of the second tube body (23); and
a packing closure (30) having a third tube body (32) formed at a center of a body (31) such that the third tube body (32) is fitted inside the second tube body (23), a fourth tube body (33) formed on an outside of the third tube body (32), a female screw (34) formed on the fourth tube body (33) and fastened to the male screw (24), and a packing (36) which has elasticity and which is disposed inside the body (31),
wherein the first tube body (22) is fitted and assembled to the branch tube (13), and medication is capable of being injected inside the T-tube (10) with a syringe by piercing the packing (36) with a syringe needle and passing the syringe needle through the third tube body (32).

2. The ventilator medication injection structure of claim 1, wherein the first tube body (22) is provided in a tapered shape having a narrow lower side, and is formed of a material having elasticity.

3. The ventilator medication injection structure of claim 1, wherein a power connector (14) to which an external power source is connected is disposed on one side of the T-tube (10), a heating wire (15) is disposed inside the T-tube (10), and the heating wire (15) is configured to be connected to the external power source and is configured to heat conditioned air.

## Patentansprüche

1. Arzneimittelinjektionsstruktur für eine Beatmungsvorrichtung, die Folgendes umfasst:
ein T-Rohr (10), das einen Rohrkörper aufweist, der sich von einem Einlass (11) zu einem Auslass (12) erstreckt, wobei auf einer Seite des Rohrkörpers ein Abzweigrohr (13) gebildet ist;
eine Abdeckung (20), die einen ersten Rohrkörper (22), der unter einem Flansch (21) gebildet ist, einen zweiten Rohrkörper (23), der im Inneren des ersten Rohrkörpers (22) gebildet ist, derart, dass der zweite Rohrkörper (23) den Flansch (21) durchdringt, und ein Außengewinde (24), das auf einer Außenumfangsfläche des zweiten Rohrkörpers (23) gebildet ist, aufweist; und
einen Dichtungsverschluss (30), der einen dritten Rohrkörper (32), der in einer Mitte eines Körpers (31) gebildet ist, derart, dass der dritte Rohrkörper (32) im Inneren des zweiten Rohrkörpers (23) eingepasst ist, einen vierten Rohrkörper (33), der auf einer Außenseite des dritten Rohrkörpers (32) gebildet ist, ein Innengewinde (34) , das auf dem vierten Rohrkörper (33) gebildet ist und am Außengewinde (24) befestigt wird, und eine Dichtung (36), die eine Elastizität aufweist und die im Inneren des Körpers (31) angeordnet ist, aufweist,
wobei der erste Rohrkörper (22) am Abzweigrohr (13) eingepasst und angebracht ist und Arzneimittel mit einer Spritze in das Innere des T-Rohrs (10) injiziert werden können, indem die Dichtung (36) mit einer Spritzennadel durchstochen wird und die Spritzennadel durch den dritten Rohrkörper (32) hindurchtritt.

2. Arzneimittelinjektionsstruktur für eine Beatmungsvorrichtung nach Anspruch 1, wobei der erste Rohrkörper (22) in einer abgeschrägten Form mit einer schmalen unteren Seite vorgesehen ist und aus einem Material gebildet ist, das Elastizität aufweist.

3. Arzneimittelinjektionsstruktur für eine Beatmungsvorrichtung nach Anspruch 1, wobei ein Stromanschluss (14), mit dem eine externe Stromquelle verbunden ist, auf einer Seite des T-Rohrs (10) angeordnet ist, ein Heizdraht (15) im Inneren des T-Rohrs (10) angeordnet ist und der Heizdraht (15) konfiguriert ist, mit der externen Stromquelle verbunden zu sein, und konfiguriert ist, aufbereitete Luft zu erwärmen.

## Revendications

1. Structure d'injection de médicament pour ventilateur comportant :
le tube en T (10) ayant un corps de tube s'étendant d'une entrée (11) à une sortie (12), avec un tube d'embranchement (13) étant formé sur un côté du corps de tube ;
un bouchon (20) ayant un premier corps de tube (22) formé sous un rebord (21), un deuxième corps de tube (23) formé à l'intérieur du premier corps de tube (22) de telle sorte que le deuxième corps de tube (23) pénètre dans le rebord (21), et une vis extérieure (24) formée sur une surface périphérique extérieure du deuxième corps de tube (23) ; et
une fermeture de garniture (30) ayant un troisième corps de tube (32) formé sur un centre d'un corps (31) de telle sorte que le troisième corps de tube (32) est inséré à l'intérieur du deuxième corps de tube (23), un quatrième corps de tube (33) formé sur un extérieur du troisième corps de tube (32), une vis intérieure (34) formée sur le quatrième corps de tube (33) et fixée à la vis extérieure (24), et une garniture (36) qui présente une élasticité et qui est disposée à l'intérieur du corps (31),
dans laquelle le premier corps de tube (22) est inséré et assemblé au tube d'embranchement (13), et un médicament peut être injecté à l'intérieur du tube en T (10) avec une seringue en perçant la garniture (36) avec une aiguille de seringue et en faisant passer l'aiguille de seringue à travers le troisième corps de tube (32).

2. Structure d'injection de médicament pour ventilateur selon la revendication 1, dans laquelle le premier corps de tube (22) est fourni avec une forme conique ayant un côté inférieur étroit, et est formé d'un matériau présentant une élasticité.

3. Structure d'injection de médicament pour ventilateur selon la revendication 1, dans laquelle un connecteur d'alimentation (14) auquel une source d'alimentation externe est raccordée, est disposé sur un côté du tube en T (10), un fil chauffant (15) est disposé à l'intérieur du tube en T (10), et le fil chauffant (15) est configuré pour être raccordé à la source d'alimentation externe et est configuré pour chauffer de l'air conditionné.
